# EUROPÄISCHE PATENTSCHRIFT

(11) **EP 1 303 302 B1**
(45) Veröffentlichungstag und Bekanntmachung des Hinweises auf die Patenterteilung: **14.10.2009**
(21) Anmeldenummer: 01965110.8
(22) Anmeldetag: 11.07.2001
(51) Int. Cl.: A61K 39/275

(54) **VERWENDUNG VON STÄMMEN DES PARAPOXVIRUS OVIS GEGEN ORGANFIBROSEN**
USE OF STRAINS OF THE PARAPOX OVIS VIRUS AGAINST ORGAN FIBROSIS
UTILISATION DE SOUCHES DU VIRUS PARAPOX OVIS CONTRE LA FIBROSE DES ORGANES

(30) Priorität: 11.07.2000 DE 10033581; 08.05.2001 DE 10122233
(43) Veröffentlichungstag der Anmeldung: 23.04.2003
(73) Patentinhaber: AiCuris GmbH & Co. KG, 42117 Wuppertal (DE)
(72) Erfinder: HIRTH-DIETRICH, Claudia, 42115 Wuppertal (DE); SCHLAPP, Tobias, 51061 Köln (DE); SIEGLING, Angela, F-75015 Paris (FR); KNORR, Andreas, 40699 Erkrath (DE); WEBER, Olaf, Woodbridge, CT 06525 (US); THEISS, Gudrun, 42329 Wuppertal (DE)
(74) Vertreter: Weller, Wolfgang
(86) Internationale Anmeldenummer: PCT/EP2001/007978
(87) Internationale Veröffentlichungsnummer: WO 2002/004019

(56) Entgegenhaltungen:
- WO-A-00/69455
- WO-A-95/22978
- WO-A-97/38724
- FRIEDMAN SCOTT L.: "Evaluation of fibrosis and hepatitis C" THE AMERICAN JOURNAL OF MEDICINE, Bd. 107, Nr. 6B, 1999, Seiten 27s-30s, XP001022493
- AHNE; MAYR: 'Poxvirus preparation CONPIND initiates production of the major inflammatory mediators IL-1alpha and TNF-alpha in human whole blood and in blood mononuclear cell cultures' COMP. IMMUN. MICROBIOL. INFECT. DIS. Bd. 20, Nr. 2, 1997, Seiten 139 - 145

## Beschreibung

Die vorliegende Erfindung betrifft die Humanverwendung von Isolaten von Parapoxvirus ovis der Stämme orf-11, Greek orf strain 176, Greek orf strain 155, New Zealand (NZ) Isolaten NZ2, NZ7 und NZ10 bei der Prophylaxe und Behandlung von Erkrankungen, die mit erhöhter Kollagenablagerung einhergehen, wobei sowohl innere Organe, wie z.B. die Leber, als auch die Haut und ihre Anhangsgebilde betroffen sein können. Insbesondere sind betroffen Leberfibrose bzw. Leberzirrhose im Gefolge von Virushepatitis oder Ethanol-induzierten Lebererkrankungen sowie Zystische Fibrose.

Ein weiterer Gegenstand der Erfindung betrifft die Verwendung von Parapoxvirus ovis D 1701 allein zur Herstellung von Arzneimitteln und pharmazeutische Zubereitungen mit vorbeugender oder heilender Wirkung auf Organfibrosen des Menschen.

Betroffen sind neben den Ausgangsstämmen auch die durch Passagierung und/oder Adaptation an bestimmte Zellen, beispielsweise WI 38, erhaltenen Abkömmlinge. Geeignet sind neben den kompletten Viren auch Teile oder Bruchstücke dieser Viren. Unter Teilen sind mittels geeigneter Vektoren beispielsweise Vaccinia in geeigneten Systemen wie Fibroblastenzellkulturen exprimierte genomische oder subgenomische Fragmente zu verstehen. Unter Bruchstücken versteht man die durch biochemische Aufreinigung wie Chromatografie erhaltenen Fraktionen oder die nach Anwendung physikalischer Methoden wie Aufschluss mit Ultraschall erhaltenen Partikel.

Es ist bekannt, dass Parapoxvirus die nonspezifische Immunreaktion von Vertebraten stimulieren kann. Baypamun®, eine Präparation von chemisch inaktiviertem Parapoxvirus ovis, Strain D1701 wird für die Prophylaxe, Metaphylaxe und Therapie von Infektionserkrankurigen sowie zur Prävention von stressinduzierten Erkrankungen bei Tieren eingesetzt.

Das Patent DE 3 504 940 A (Mayr, Anton) lehrt die günstige Winkung von Baypamun® bei Zuständen wie der Immuninsuffizienz durch energiereiche Bestrahlung, Chemotherapie, AIDS, Immunsuppression, altersbedingten Schäden sowie detoxifizierende Effekte, nicht aber die unmittelbare Reduktion einer Leberfibrose. Weiterhin lehrt DE 3 504 940, dass Baypamun® als Adjunkt die Wirksamkeit einer Tumortherapie unterstützt, und dass es Neugeborene vor durch unzureichende maternale Immunabwehr hervorgerufenen Erkrankungen schützt.

Dokument WO 95/22978 beschreibt die Verwendung einer Zusammensetzung, die zumindest zwei oder mehr Parapoxvirusstämme enthält, als Paramunitätsinduktor, d.h. zur unspezifischen Stimulierung des Immunsystems.

Dokument WO 97/38724 beschreibt die Verwendung einer als "Conpind" bezeichneten Zusammensetzung, die aus den beiden Virusstämmen Parapoxvirus ovis D1701 und Avipoxvirus gallinae besteht, als Paramunitätsinduktor.

Ausgehend vom vorliegenden Stand der Wissenschaft wurde jetzt überraschenderweise gefunden, dass die Gabe inaktivierter Parapoxviren die Leberfibrose verringern bzw. verhindern kann. Diese Wirkung wurde in Tiermodellen bei der Tetrachlorkohlenstoff-induzierten Leberfibrose gesunden, die auf einer toxischen Leberschädigung beruht, und bei der durch artfremde Serum induzierten Leberfibrose, bei der es nicht zu einer Leberentzündung kommt. Überraschend ist außerdem das Ausmaß des therapeutischen Effekts: Die in der Leberfibrose überschießende Kollagen-Produktion wird im Tetrachlorkohlenstoff-Modell zu 60 %, im Serum-Modell fast vollständig gehemmt. In Übereinstimmung mit diesen Ergebnissen aus Langzeitexperimenten konnte nach akuter Gabe von Tetrachlorkohlenstoff jetzt gezeigt werden, dass Baypamun® und die aus den oben genannten Stämmen von Parapoxvirus ovis erhaltenen Zubereitungen die Transformation der hepatischen Sternzellen zum kollagenproduzierenden Myofibroblasten-Typ hemmen.

Leberfibrose bzw. Leberzirrhose kann durch unterschiedliche Noxen wie beispielsweise virale Infektionen und Alkoholabusus ausgelöst werden, doch münden die unterschiedlichen Pathomechanismen in eine gemeinsame Endstrecke, die Kollagen-Produktion, ein. Wie die tierexperimentellen Ergebnisse aus den oben geschilderten nicht-infektiösen Modellen zeigen, verhindert die Gabe inaktivierter Parapox-Viren überraschenderweise die Kollagenablagerung unabhängig von der auslösenden Noxe. Parapox-Viren eröffnen damit ein neuartiges Therapieprinzip zur Einwirkung auf die allen zur Fibrose führenden Erkrankungen gemeinsame Endstrecke.

Dieser Effekt lässt eine besonders effektive Therapie auch der viral induzierten Leberfibrose mit Zubereitungen von Parapoxviren erwarten, da für solche Zubereitungen zusätzliche immunstimulatorische Wirkungen bekannt sind.

Die jetzt aufgefundene starke antifibrotische Wirkung von Baypamun® öffnet die Möglichkeit, Baypamun® oder Zubereitungen von NZ2 als Referenz Standard für die Beurteilung der antifibrotischen Effekte in Assays zur Auffindung antifibrotischer Substanzen einzusetzen.

Inaktivierte Parapox-Viren oder deren Abkömmlinge und Zubereitungen erhalten aus den oben genannten Stämmen besitzen also ein universelles antifibrotisches Wirkspektrum und sind daher nicht nur für die Prophylaxe und Therapie fibrotischer Erkrankungen der Leber, sondern auch bei fibrotischen Erkrankungen anderer Organe, wie beispielsweise der Lunge, des Pankreas , des Herzens und der Haut geeignet. Besonders bevorzugt ist die Verwendung von Isolaten von Parapoxviren bei der Prophylaxe und Behandlung von Leberfibrose und Leberzirrhose.

Je nach klinischer Fragestellung wird das Therapeutikum auf der Basis von Parapoxvirus systemisch, also beispielsweise intramuskulär, subcutan, intraperitoneal, intravenös, per os, per inhalationem oder lokal appliziert. Das Parapoxvirus liegt dabei entweder aufgereinigt und lyophilisiert vor und wird unmittelbar vor der Applikation in einem geeigneten Lösungsmittel suspendiert oder es liegt in einer anderen geeigneten Formulierung vor oder es liegt in einer magensaftresistenten oder einer anderen oralen Applikationsform vor.

Mehrere Applikationen oder Langzeitbehandlung nach Zeitschemen, die den Erfordernissen der klinischen Fragestellung entsprechen, können dabei notwendig sein.

Die vorliegende Erfindung betrifft die Verwendung von Isolaten von Parapoxviren zur Herstellung von Arzneimitteln mit vorbeugender oder heilender Wirkung auf Organfibrosen des Menschen. Isolate von Parapoxviren, erhalten aus den Stämmen D1701, orf-11, Greek orf strain 176, Greek orf strain 155, sowie den New Zealand (NZ) Stämmen zur Herstellung von Arzneimitteln mit vorbeugender oder heilender Wirkung auf Organfibrosen des Menschen. Bevorzugt verwendet werden New Zealand (NZ) Stämme, die Stämme NZ2, NZ7 und NZ10 zur Herstellung von Arzneimitteln mit vorbeugender oder heilender Wirkung auf Organfibrosen des Menschen, wobei der Stamm NZ2 besonders bevorzugt ist. Darüber hinaus können die vorstehend beschriebenen Parapox Viren durch Passagierung oder Adaptation an geeigneten Zellen modifiziert werden und solche durch Passagierung oder Adaptation erhaltenen Parapox Viren zur Herstellung von Arzneimitteln mit vorbeugender oder heilender Wirkung auf Organfibrosen des Menschen verwendet werden, wobei zur Passagierung oder Adaptation beispielsweise humane Zellen wie WI-38, MRC-5, bovine Zellen, wie BK-K13A47/Reg oder MDBK und ovine Zellen, wie MDOK verwendet werden können. Auch Teile oder Bruchstücken der vorstehend genannten Parapox Viren können zur Herstellung von Arzneimitteln mit vorbeugender oder heilender Wirkung auf Organfibrosen des Menschen verwendet werden. Unter Teilen wird verstanden mittels geeigneter Vektoren wie Vacciniaviren in geeigneten Systemen wie Fibroblastenzellkulturen exprimierte genomische oder subgenomische Fragmente und unter Bruchstücken die durch biochemische Aufreinigung wie Chromatografie erhaltenen Fraktionen der exprimierten oder physikalisch, beispielsweise durch Einwirkung von Ultraschall, aufgeschlossenen viralen Partikel. Ein weiterer Gegenstand der Erfindung ist die Verwendung der vorstehend beschriebenen Stämme von Parapoxvirus ovis oder der daraus wie vorstehend beschrieben erhaltenen Modifikate in Kombination mit anderen Mitteln zur Herstellung von Arzneimitteln und pharmazeutischen Zubereitungen mit vorbeugender oder heilender Wirkung auf Organfibrosen des Menschen sowie die Verwendung von Baypamun® allein oder in Kombination mit anderen Mitteln zur Herstellung von Arzneimitteln und pharmazeutischen Zubereitungen mit vorbeugender oder heilender Wirkung auf Organfibrosen des Menschen. Ein bevorzugter Gegenstand der Erfindung ist die Verwendung der vorstehend beschriebenen Stämme von Parapoxvirus ovis oder der daraus wie vorstehend beschrieben erhaltenen Modifikate in Kombination mit anderen Mitteln in einer Formulierung für die orale Applikation, z. B. in magensaftresistenten Kapseln.

Weiter ist Gegenstand der Erfindung die Verwendung von Parapox ovis D 1701 oder Zubereitungen von NZ2 als Referenz für die Beurteilung der antifibrotischen Effekte in Assays zur Auffindung antifibrotischer Substanzen.

Der hier beispielhaft genannte Parapoxvirus ovis NZ-2 wurde am 10. Juli 2001 bei der European Collection of Cell Cultures, Centre for Applied Microbiology and Research, Porton Down, Salisbury, Wiltshire, SP4 OJG, United Kingdom hinterlegt. Die Hintelegungsnummer lautet 0 107 1006.

### Beispiel 1

### Wirkung von Parapoxvirus ovis, Stamm D 1701, Baypamun®

### Methodik

Baypamun® Trockensubstanz, eine Präparation gewonnen aus chemisch inaktiviertem Parapoxvirus Ovis, Strain D 1701, wurde nach Vorschrift mit Aqua pro injektione gelöst (Titer bezogen auf die TCID50 (50 % tissue culture infective dose) ca. 10⁷ pro ml).

Als Placebo-Lösung wurde den Tieren der Kontrollgruppen eine Polygeline-Lösung mit dem gleichen Proteingehalt wie in der Baypamun® - Lösung verabreicht.

Pro Tier und Applikation wurden 0,5 ml Lösung i.p. verabreicht. Die Applikation wurde drei mal pro Woche, jedoch nie an aufeinanderfolgenden Tagen durchgeführt.

Baypamun®, wurde in zwei Tiermodellen mit unterschiedlicher Genese der Fibrose getestet, dem Tetrachlokohlenstoff-Modell und dem Schweineserum-Modell.

Die chronische Behandlung mit Tetrachlorkohlenstoff ist eine Standardmethode zur experimentellen Auslösung einer Leberfibrose mit anschließender Zirrhose (McLean EK, McLean AEM, Sutton PM. Instant cirrhosis. Br. J Exp. Pathol. 1969; 50: 502-506). Es ist allgemein als Modell der menschlichen Leberfibrose und -zirrhose anerkannt. Es wurden weibliche Sprague-Dawley Ratten verwendet. Zur maximalen Induktion des mikrosomalen Metabolismus von Tetrachlorkohlenstoff erhielten die Tiere 1 g/l Isoniazid mit dem Trinkwasser bereits eine Woche vor dem Beginn der Behandlung. Tetrachlorkohlenstoff wurde oral jeden fünften Tag in einer Dosis von 0,1 ml/100g Körpergewicht (Tetrachlorkohlenstoff : Mineralöl = 1 : 1) gegeben. Die Tiere wurden nach siebenwöchiger Behandlung getötet und untersucht. Die Behandlung mit Baypamun® wurde parallel zur Tetrachlorkohlenstoff-Behandlung durchgeführt.

Die Behandlung mit heterologem Serum, z.B. Schweineserum bei Ratten, ist ebenfalls eine häufig in der Literatur angewendete Methode zur Auslösung einer Leberfibrose mit anschließender Zirrhose, die im Gegensatz zu anderen Modellen nur eine minimale Schädigung und Entzündung der Leberzparenchymzellen hervorruft (Bhunchet, E. and Wake, K. (1992): Role of mesenchymal cell populations in porcine serum-induced rat liver fibrosis. Hepatology 16: 1452-1473). Weibliche Sprague Dawley Ratten wurden 2 x wöchentlich mit 0,.5 ml/Tier sterilem Schweineserum (Sigma) i.p. behandelt, Kontrolltiere mit steriler physiologischer Kochsalzlösung (2 x wöchentlich 0,5 ml/Tier i.p.).Die Behandlung mit Baypamun® erfolgte parallel zur Schweineserum-Behandlung, jedoch nie am selben Tag. Nach siebe Wochen Behandlung wurden die Tiere getötet und die Lebern zur Quantifizierung des Kollagengehalts entnommen.

Für die histologische Untersuchung des Lebergewebes wurden standardisierte transverse Gewebezylinder (etwa 10 x 2 mm) aus dem rechten Vorderlappen der Leber gestanzt. Gefrierschnitte wurden für die Detektion von durch Leberfibrose hervorgerufenem Narbenkollagen mit 0,1 %iger Pikrosiriusrot-Lösung gefärbt.

Fast Green wurde als Gegenfärbung zur Kontrastverstärkung eingesetzt. Das Ausmaß der Leberfibrose wurde in jedem Schnitt als prozentualer Anteil der Pikrosiriusrotgefärbten Fläche an der gesamten Meßfläche bestimmt. Die Parameter der videomikroskopischen Farbdetektion wurden standardisiert und im gesamten Experiment konstant gehalten. 64 Felder eines standardisierten Rasters von 31 mm² wurden bei 50-facher Endvergrößerung ausgemessen.

Zur Quantifizierung des Ausmaßes der Transformation von hepatischen Sternzellen (HSC; auch Itozellen oder Vitamin-A-Speicherzellen) nach akuter Behandlung von Ratten mit Tetrachlorkohlenstoff wurden die α-Glattmuskelaktin-positiven Zellen immunhistochemisch detektiert. Die α-Glattmuskelaktin-positive Fläche wurde bei 200-facher Endvergrößerung in jedem Schnitt in jeweils 16 zentrilobulären Feldern von 248 x 180 µm festgestellt. Die Transformation von HSC in kollagen- und wachstumsfaktorproduzierende myofibroblastartige Zellen ist bekanntlich der entscheidende Schritt bei der Auslösung der Leberfibrose. Daher sind transformierte HSC ein Frühindikator fibrogener Aktivität in der Leber.

Für die halbautomatische Morphometrie wurde ein Leica Quantimed 500MC (Leica Deutschland) eingesetzt.

Zur OH-Prolin-Bestimmung wurden je 50-100 mg Lebergewebe getrocknet und mit 6N HCl ca. 17 Stunden gekocht. Nach Verdampfen der Säure im Vakuum-Trockenschrank wurde der Rückstand mit 5 ml Aqua dest, gelöst und filtriert. 200 µl der filtrierten Lösung wurden mit 200 µl Ethanol und 200 µl Oxidationslösung (7%ige wässrige Chloramin T Hydrat-Lösung 1 : 4 mit Acetat-Citrat-Puffer pH 6,0 verdünnt) 25 min bei Zimmertemperatur inkubiert. Danach wurden 400 µl Ehrlich's Reagenz (12 g 4-Dimethylaminobenzaldehyd in 20 ml Ethanol + 2,74 ml konzentrierte Schwefelsäure in 20 ml Ethanol) zugegeben. Nach 3 Stunden Inkubation bei 35°C wurde die Absorption bei 573 nm gemessen. Für die Eichreihe wurden wässrige OH-Prolin (Sigma)-Lösungen verwendet. Der OH-Prolin-Gehalt der Leberproben wurde in mg pro g Leber-Trockengewicht berechnet.

Zur Verfolgung der Entstehung von reaktiven Sauerstoffradikalen wurde die Konzentration an reduziertem α-Tocopherol (α-TOC), einem Radikalfänger, in der Leber und die Aktivität des radikalsensitiven Enzyms 7-Ethoxyresorufin-Deethylase (EROD) im Serum gemessen. Beide Parameter sind charakteristischerweise bei der Tetrachlorkohlenstoffvergiftung herunterreguliert und erlauben eine Abschätzung des Schweregrades der oxidativen Gewebeschädigung.

Der Leberstatus der Tiere wurde durch Messung einiger Standardserumparameter bestimmt:

Alaninaminotransferase (ALT), alkalische Phosphatase (AP), Aspartataminotransferase (AST), γ-Glutamyltransferase (GGT), Glutamatdehydrogenase (GLDH), Gesamtbilirubin (TBIL).

### Ergebnisse:

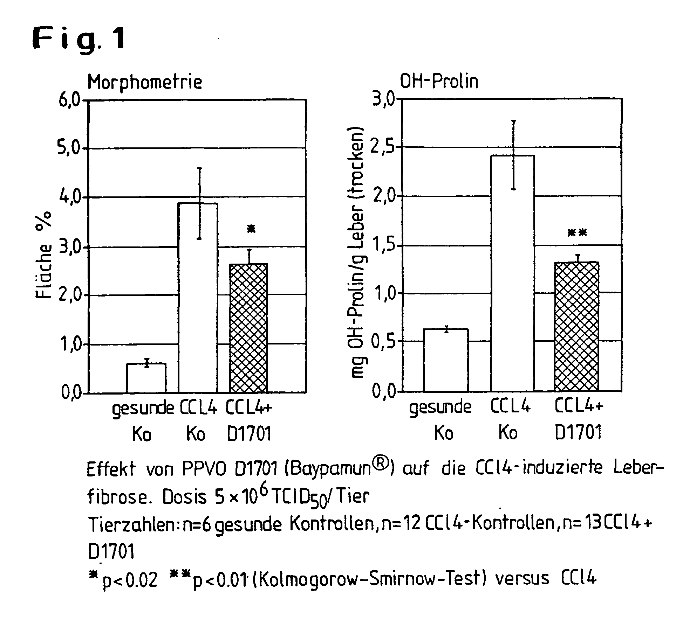

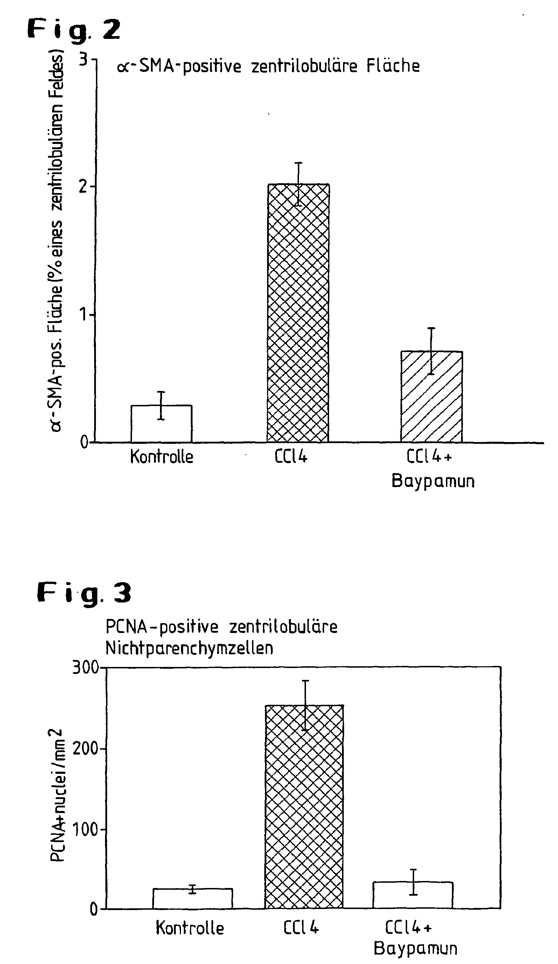

Die Behandlung mit Baypamun^{®} vermindert signifikant das Ausmaß der fibrotischen Degeneration der Lebern von tetrachlorkohlenstoffbehandelten Ratten (Abb. 1). Daneben ist eine fast komplette Hemmung der HSC-Transformation zu beobachten (Abb. 2). Die Anzahl proliferierender Nichtparenchymzellen in den - Lebern Baypamun^{®}-behandelter Tiere ist deutlich verringert (Abb. 3). Nichtparenchymzellen schließen HSC und die gleichfalls an der Fibrogenese beteiligten Kupferzellen ein.

Die Serumindikatoren für eine hepatozelluläre Schädigung wie ALT, AP, AST, GGT, GLDH und TBIL (Tabelle 1) zeigen eine Tendenz zur Normalisierung.

Die gleiche Verminderung der EROD- und α-Tocopherolkonzentrationen in der Kontrollgruppe und der mit Baypamun^{®} behandelten Gruppe belegt die Anwesenheit von toxischen reaktiven Sauerstoffradikalen durch die Tetrachlorkohlenstoffvergiftung in beiden Gruppen (Tabelle 1 und 2). Daher kann ein "antifibrotischer" Effekt von Baypamun^{®} durch eine detoxifizierende Wirkung ausgeschlossen werden.

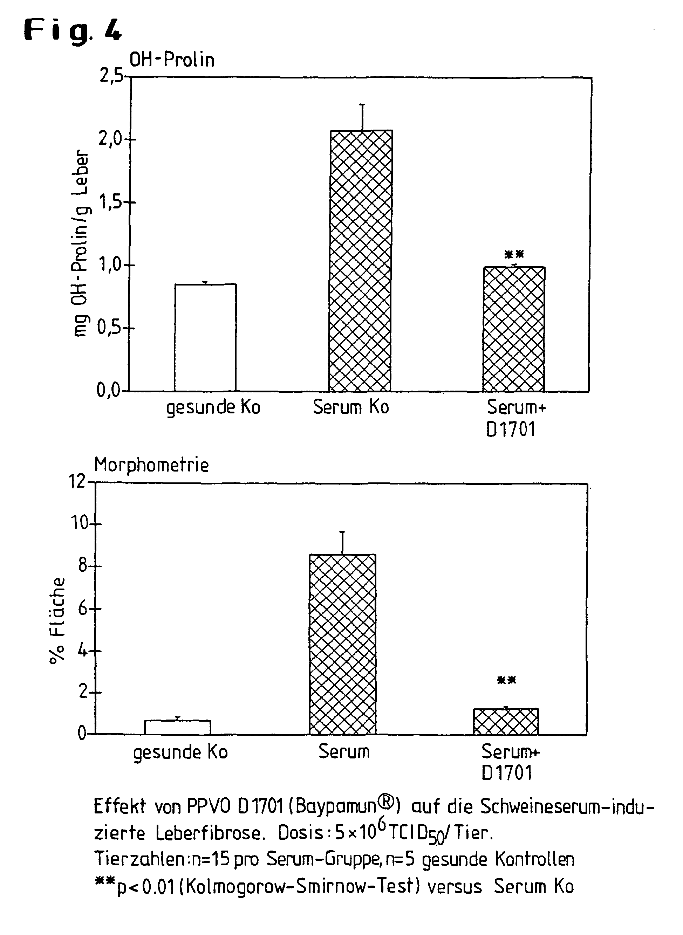

Im Serum-Modell zeigt Baypamun® eine nahezu vollständige Unterdrückung der Fibrose(Abb 4): Sowohl der Hydroxyprolin-Gehalt als auch die mit Siriusrot anfärbbare Fläche der Lebern liegt bei den mit Baypamun® behandelten Ratten fast auf dem Niveau der gesunden Kontrolltiere, während sie bei den Serum-behandelten Kontrollratten um ein Mehrfaches erhöht ist. Der durch die Schweineserumbehandlung induzierte Anstieg des Kollagengehalts beträgt in der Baypamun-Gruppe nur 10 % des entsprechenden Wertes in der Kontroll-Gruppe.

### Beispiel 2:

### Parapoxvirus ovis, Strain NZ 2

### Methodik:

NZ2-Virus wurde in Wannenstapeln vermehrt. Hierzu wurden BK-Klon3A Zellen in Zellkulturschalen (37°C) 3 bis 5 Tage in EMEM 2 gr + 10 % FCS angezogen bis der Zellrasen 90 bis 100 % konfluent war. Pro Wannenstapel wurden 4 Zellkulturschalen als Inokulum verwendet und mit Medium (EMEM 2 gr + 10% FCS) auf ein Volumen von 2,5 Liter aufgefüllt. Nach 3 bis 5 Tagen Inkubation bei 37°C (90 bis 100 % konfluenter Zellrasen) wurde das Medium gegen EMEM 2 g ohne Serumzusatz ausgetauscht und die Kultur mit NZ2-Virus (MOI 0,001 bis 0,01) infiziert.

Nach Erreichen von 100% CPE (Inkubation 37°C, ca. 7 bis 8 Tage) wurde das Virus geerntet. Hierzu wurde die Virussuspension in sterile Mediensäcke abgefüllt und bei -80°C eingefroren. Anschließend wurde die Suspension bei 37°C im Brutraum aufgetaut und durch eine Tiefenfiltration (Porengröße 5 µm) zellbefreit. Hierauf wurde die Virussuspension durch Ultrafiltration (100 kDa cutoff) um den Faktor 20 bis 40 aufkonzentriert. Alternativ kann das Virus durch Ultrazentrifugation (Ti45 30.000 rpm, 4°C, 60 Min) aufkonzentriert werden.

Der durch die Aufkonzentrierung erreichte Titer des in der Suspension enthaltenen Virus wurde durch Titration auf BK-Klon3A Zellen bestimmt. Nachdem der Virustiter auf 6,0 mittels EMEM Medium ohne FCS eingestellt wurde, wurde das Virus 2h bei 58°C hitzeinaktiviert. Die Inaktivierung wurde durch eine Inaktivierungskontrolle auf BK-Klon3A Zellen kontrolliert.

Parapoxvirus ovis, Strain NZ2 wurde in dem bereits in Beispiel 1 verwendeten Modell der Schweineserum-induzierten Leberfibrose bei Ratten untersucht:

Weibliche Sprague Dawley Ratten wurden 2 x wöchentlich mit 0,5 ml/Tier sterilem Schweineserum (Sigma) i.p. behandelt, Kontrolltiere mit steriler physiologischer Kochsalzlösung (2 x wöchentlich 0,5 ml/Tier i.p.). Strain NZ 2 wurde 3 x wöchentlich in den Dosierungen 1,5 x 10⁵ bzw. 5,0 x 10⁵ TCID₅₀/Tier i.p. verabreicht (Applikationsvolumen: 0,5 ml/Tier). Für die untere Dosis wurde das Ausgangsmaterial mit Zellkulturmedium (Minimum Essential Medium Eagle, Sigma) verdünnt. Kontrolltiere wurden mit Zellkulturmedium behandelt. Die Behandlung mit Strain NZ 2 erfolgte parallel zur Serum-Behandlung, jedoch nie am selben Tag. Nach 7 Wochen Behandlung wurden die Tiere getötet, die Lebern entnommen und die Leberfibrose sowohl morphometrisch als auch über den OH-Prolin-Gehalt quantifiziert. Die Methodik hierzu ist bereits in Beispiel 1 beschrieben.

### Ergebnisse:

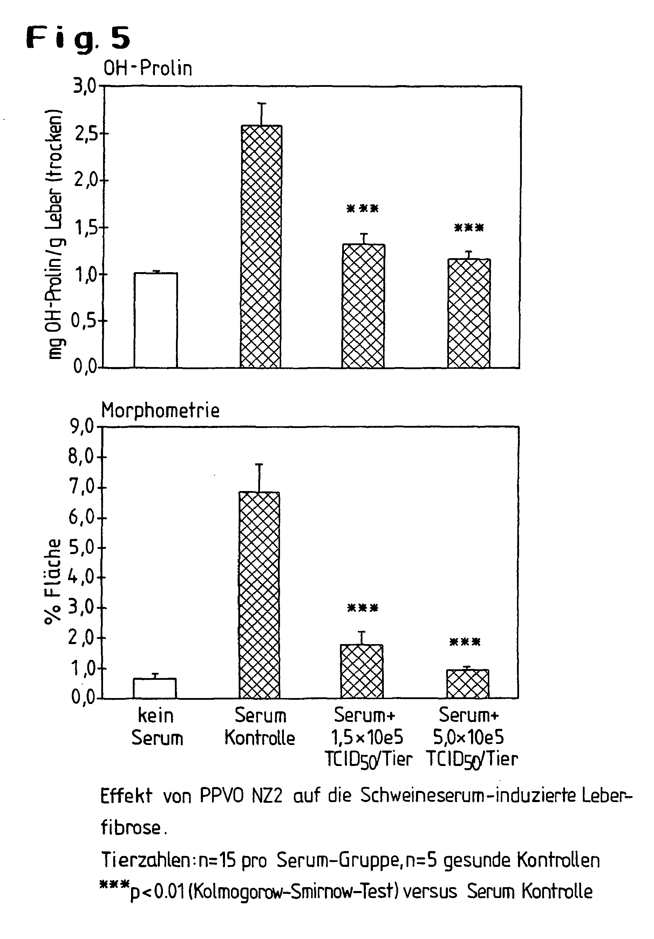

Die Ergebnisse der Kollagenbestimmungen sind in Abb. 5 dargestellt. Überraschenderweise kann die Behandlung mit Strain NZ 2 das Entstehen der Leberfibrose unterdrücken: Die Serum-behandelten Kontroll-Tiere zeigen im Vergleich zu gesunden Tieren eine deutliche Erhöhung des OH-Prolin-Gehalts und des Siriusrot-färbbaren Kollagens. Dieser Anstieg wird durch NZ 2 dosisabhängig reduziert. Überraschend ist auch das Ausmaß des Effekts: Die Dosis 5 x 10⁵ TCID₅₀ reduziert den Anstieg des Kollagengehalts in der Leber auf weniger als 10 % des Kontrollwertes. Eine qualitative Bewertung der histologischen Präparate ergab, dass der Anteil der Tiere mit Kollagen-Septen von 93 % (14/15) auf 33 % (5/15) in der 1.5 x 10⁵ TCID₅₀-Gruppe und auf 0 % in der 5,0 x 10⁵ TCID₅₀-Gruppe gesenkt wird.

### Beispiel 3

### Antifibrotische Wirkung von PPVO nach oraler Verabreichung.

PPVO wurde als trockenes Lyophilisat in magensaftresistenten Kapseln (Elanco, Indianapolis, USA) formuliert.

Vier Gruppen von je sechs Versuchstieren wurden wie folgt behandelt: Eine Kontrollgruppe (Gruppe 1) erhielt lediglich eine magensaftresistente Kapsel (ohne PPVO) oral verabreicht, und zusätzlich sterile Kochsalzlösung (0,5 ml/Tier) i.p. injiziert. Bei Gruppe 2 wurde eine magensaftresistente Kapsel (ohne PPVO) zusammen mit 0,5 ml Tetrachlorkohlenstoff Tier oral verabreicht und 0,5 ml physiologische Kochsalzlösung i.p. injiziert. Tiere der Gruppe 3 erhielten wiederum eine magensaftresistente Kapsel (ohne PPVO) zusammen mit 0,5 ml Tetrachlorkohlenstoff / Tier oral verabreicht. Die Tiere der Gruppe 3 erhielten außerdem PPVO D1701 (Dosis: 5 x 10⁶ TCID₅₀ / Tier) in 0,5 ml Aqua pro injektione i.p. injiziert. Gruppe 4 erhielt PPVO D1701 (Dosis: 5 x 10⁶ TCID₅₀ / Tier, in einer magensaftresistenten Kapsel formuliert) zusammen mit 0,5 ml Tetrachlorkohlenstoff oral verabreicht. Tiere der Gruppe 4 erhielten außerdem 0,5 ml sterile Kochsalzlösung i.p. injiziert.

Nach 48 Stunden wurden die Lebern entnommen und in repräsentativen Gewebeschnitten die α-Glattmuskelaktin (α-SMA) -positive zentrilobuläre Fläche in % der gesamten Messfläche immunhistochemisch für jedes Tier bestimmt (Johnson S J, Hines JE, Burt AD. Phenotypic modulation of perisinusoidal cells following acute liver injury: a quantitative analysis. Int. J Exp. Path. 1992; 73: 765-772). Dieser Wert ist ein Maß für die Transformation der Lebersternzellen.

Es wurden 2 Versuchsreihen nach obiger Beschreibung durchgeführt. Die Versuchsergebnisse der ersten Versuchsreihe sind in Tabelle 3, die Ergebnisse der 2. Versuchreihe sind in Tabelle 4 wiedergegeben.

In Versuchsreihe 1 ist der Anteil der α-SMA positiven zentrilobulären Fläche in Lebergewebe von Tieren, die PPVO D1701 i.p. appliziert bekamen (Gruppe 3) unerklärlicherweise (und entgegen der sonstigen experimentellen Erfahrung) höher als bei Tieren, die kein PPVO verabreicht bekamen. Aus diesem Grunde wurde die Versuchsreihe 2 als Wiederholungsexperiment durchgeführt.

In beiden Versuchsreihen wurde übereinstimmend und überraschenderweise nach oraler Gabe von PPVO D1701 (jeweils Gruppe 4) eine ca. 50 %ige Hemmung der Transformation gegenüber der Kontrollgruppe (jeweils Gruppe 2) festgestellt. In der 2. Versuchsreihe ist die Hemmung der Transformation nach oraler Gabe von PPVO D1701 (Gruppe 4) ähnlich effektiv wie bei peritonealer Verabreichung von PPVO D1701 (Gruppe 3).

Aus diesen Ergebnissen lässt sich ableiten, dass PPVO seine antifibrotische Wirkung auch nach oraler Gabe entfaltet.

**Tabelle 1**

| **Einfluß von Baypamun^{®} auf Serumparameter des Leberstatus und Indikatoren einer Intoxikation** | | | | | | | |
|---|---|---|---|---|---|---|---|
| | ***ALT*** | ***AST*** | ***AP*** | ***GGT*** | ***GLDH*** | ***TBIL*** | ***EROD*** |
| | U/l | U/l | U/l | U/l | U/l | µmol/l | nmol g x min |
| ***Kontrolle*** | 49.1 | 45.7 | 162.8 | 0.8 | 12.5 | 1.6 | 0.30 |
| *SEM* | 3.7 | 8.7 | 14.76 | 0.2 | 7.9 | 0.2 | 0.02 |
| | | | | | | | |
| ***Tetrachlorkohlenstoff*** | 95.6 | 92.4 | 392.7 | 6.1 | 37.1 | 2.9 | 0.15 |
| *SEM* | 14.7 | 14.1 | 43.0 | 1.3 | 14.8 | 0.3 | 0.01 |
| | | | | | | | |
| *T*.+ **Baypamun^{®}** | 72.0 | 65.9 | 329.5 | 4.5 | 18.2 | 1.9 | 0.17 |
| *SEM* | 9.9 | 9.1 | 26.9 | 0.8 | 4.0 | 0.2 | 0.03 |

| | | | | | | | |
|---|---|---|---|---|---|---|---|
| ALT : Alaninaminotransferase GLDH : Glutamatdehydrogenase AST : Aspartataminotransferase TBIL : Gesamtbilirubin AP : alkalische Phosphatase EROD : 7-Ethoxyresorufindeethylase GGT : γ-Glutamyltransferase | | | | | | | |

**Tabelle 2**

| **Einfluß von Baypamun^{®}** | |
|---|---|
| **auf Leber-α-Tocopherol** | |
| | α-*Tocopherol* nmol / g Gewebe |
| ***Kontrolle*** | 73.1 |
| *SEM* | 2.2 |
| | |
| ***Tetrachlorkohlenstoff*** | 35.7 |
| *SEM* | 2.3 |
| | |
| ***T*. + Baypamun^{®}** | 38.5 |
| *SEM* | 6.1 |

**Taballe 3**

| Einfluss von PPVO nach intraperitonealer oder oraler Gabe auf die Transformation von Leberstemzellen nach Gabe einer fibrogenen Dosis Tetrachlorkohlenstoff. (Versuchsreihe 1) | | | |
|---|---|---|---|
| **Fibrose** | **Applikation** | **DOSIS** | **α-SMA (%)** |
| Gruppe 1 Intakt | Leerkapsel oral + Aqua pro injektione i.p. | ----- | 0.28 ± 0.04 |
| Gruppe 2 Tetrachlorkohlenstoff | Leerkapsel oral + Aqua pro injektione i.p. | ----- | 2.76 ± 0.79 |
| Gruppe 3 Tetrachlorkohlenstoff | PPVO in Aqua pro injektione i.p. + Leerkapsel oral | 5 x 10⁶TCID₅₀/ Tier | 5.05 ± 2.00 |
| Gruppe 4 Tetrachlorkohlenstoff | PPVO in Kapsel oral + Aqua pro injektione i.p. | 5x10⁶ TCID₅₀/ Tier | 1.46 ± 0.34 |

**Tabelle 4**

| Einfluss von PPVO nach intraperitonealer oder oraler Gabe auf die Transformation von Lebersternzellen nach Gabe einer fibrogenen Dosis Tetrachlorkohlenstoff. (Versuchsreihe 2) | | | |
|---|---|---|---|
| **Fibrose** | **Applikation** | **DOSIS** | **α-SMA (%)** |
| Intakt Gruppe 1 | Leerkapsel oral + Aqua pro injektione i.p. | ----- | 0.20 ± 0.04 |
| Tetrachlorkohlenstoff Gruppe 2 | Leerkapsel oral + Aqua pro injektione i.p. | ----- | 3.18 ± 0.56 |
| Tetrachlorkohlenstoff Gruppe 3 | PPVO in Aqua pro injektione i.p. + Leerkapsel oral | 5 x 10⁶ TCID₅₀/Tier | 1.22 ± 0.35 |
| Tetrachlorkohlenstoff Gruppe 4 | PPVO in Kapsel oral + Aqua pro injektione i.p. | 5 x 10⁶ TCID₅₀/Tier | 1.55 ± 0.34 |

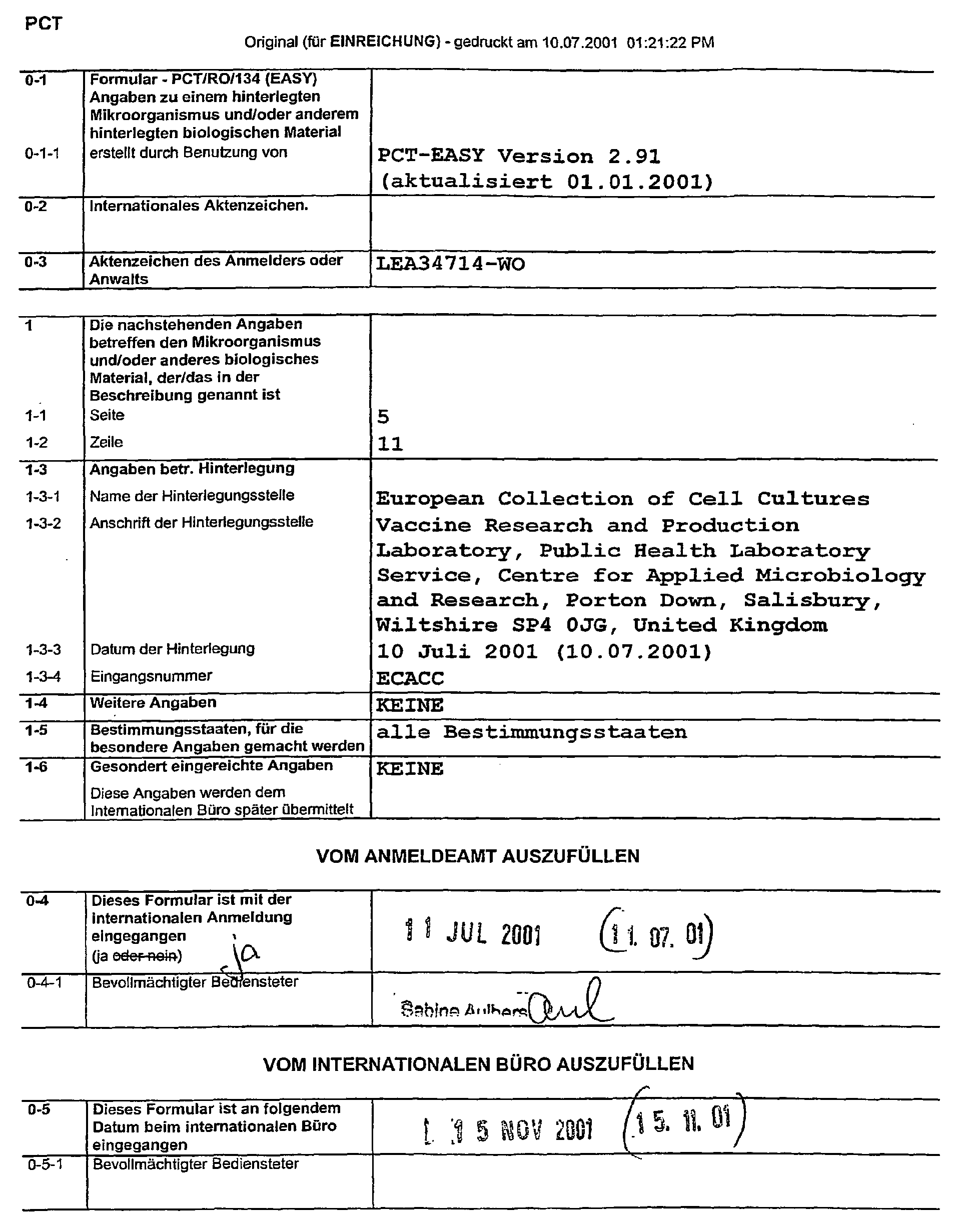

## Patentansprüche

1. Verwendung von Isolaten von Parapoxviren der Stämme orf-11, Greek orf strain 176, Greek orf strain 155, oder der New Zealand (NZ) Stämme NZ2, NZ7 und NZ10 zur Herstellung von Arzneimitteln mit vorbeugender oder heilender Wirkung auf Organfibrosen des Menschen.

2. Verwendung nach Anspruch 1, wobei solche Parapoxviren verwendet werden, die durch Passagierung oder Adaptation an geeignete Zellen modifiziert wurden.

3. Verwendung nach Anspruch 2, wobei zur Passagierung oder Adaptation humane Zellen wie WI-38, MRC-5, bovine Zellen, wie BK-K13A47/Reg oder MDBK und ovine Zellen, wie MDOK verwendet werden.

4. Verwendung nach einem der Ansprüche 1 - 3 in Kombination mit anderen Mitteln zur Herstellung von Arzneimitteln und pharmazeutischen Zubereitungen mit vorbeugender oder heilender Wirkung auf Organfibrosen des Menschen.

5. Verwendung von Parapox ovis D 1701 allein zur Herstellung von Arzneimitteln Verwendung von Parapox ovis D 1701 allein Herstellung von Arzneimitteln und pharmazeutischen Zubereitungen mit vorbeugender oder heilender Wirkung auf Organfibrosen des Menschen.

6. Verwendung von Parapox ovis D 1701 oder Zubereitungen von NZ2 als Referenz-Standard für die Beurteilung antifibrotischer Effekte in Assays zur Auffindung antifibrotischer Substanzen.

7. Verwendung von Parapox ovis D 1701 entsprechend Anspruch 5, wobei die pharmazeutischen Zubereitungen und Arzneimittel für die orale Verabreichung geeignet sind.

8. Verwendung einer Präparation von chemisch inaktiviertem Parapoxvirus ovis, Strain D 1701 allein zur Herstellung einer pharmazeutischen Zubereitung mit vorbeugender oder heilender Wirkung auf Organfibrosen des Menschen.

## Claims

1. Use of isolates of parapoxviruses of the strains orf-11, Greek orf strain 176, Greek orf strain 155, or the New Zealand (NZ) strains NZ2, NZ7 and NZ10 for producing medicaments comprising a preventive or curative effect on organ fibroses in humans.

2. Use according to claim 1, whereby such parapoxviruses are used which have been modified by passaging or adaption to suitable cells.

3. Use according to claim 2, whereby human cells such as WI-38, MRC-5, bovine cells, such as BK-K13A47/Reg or MDBK or ovine cells, such as MDOK, are used for passaging or adaption.

4. Use according to any of claims 1 to 3 in combination with other means for producing medicaments and pharmaceutical preparations comprising a preventive or curative effect on organ fibroses in humans.

5. Use of Parapox ovis D 1701 alone for producing medicaments and pharmaceutical preparations comprising a preventive or curative effect on organ fibroses in humans.

6. Use of Parapox ovis D 1701, or preparations of NZ2, as a reference standard for assessing antifibrotic effects in assays for identifying antifibrotic substances.

7. Use of Parapox ovis D 1701 according to claim 5, whereby the pharmaceutical preparations and medicaments are suitable for oral administration.

8. Use of a preparation of chemically inactivated Parapoxvirus ovis, strain D 1701, alone for producing a pharmaceutical preparation comprising a preventive or curative effect on organ fibroses in humans.

## Revendications

1. Utilisation d'isolats de virus parapox ovis issus des souches orf-11, Greek orf strain 176, Greek orf strain 155 ou des souches New Zealand (NZ) NZ2, NZ7 et NZ10 pour la fabrication de médicaments à l'action préventive ou curative de la fibrose des organes chez l'homme.

2. Utilisation selon la revendication 1, des virus parapox ovis qui ont été modifiés par passage ou adaptation sur des cellules appropriées étant utilisés.

3. Utilisation selon la revendication 2, des cellules humaines comme WI-38, MRC-5, des cellules bovines comme BK-K13A47/Reg ou MDBK ou des cellules ovines comme MDOK ayant été utilisées pour le passage ou l'adaptation.

4. Utilisation selon l'une quelconque des revendications 1 à 3 en combinaison avec d'autres agents pour la fabrication de médicaments et de préparations pharmaceutiques à l'action préventive ou curative de la fibrose des organes chez l'homme.

5. Utilisation de parapox ovis D 1701 seul pour la fabrication de médicaments et de préparations pharmaceutiques à l'action préventive ou curative de la fibrose des organes chez l'homme.

6. Utilisation de parapox ovis D 1701 ou de préparations de NZ2 comme norme de référence pour l'évaluation des effets antifibrotiques dans des dosages afin de détecter les substances antifibrotiques.

7. Utilisation de parapox ovis D 1701 selon la revendication 5, les préparations pharmaceutiques et les médicaments étant adaptés pour l'administration orale.

8. Utilisation d'une préparation de virus parapox ovis rendu chimiquement inactif, Strain D1701, pour la fabrication d'une préparation pharmaceutique à l'action préventive ou curative de la fibrose des organes chez l'homme.
